# EUROPÄISCHE PATENTSCHRIFT

(11) **EP 4 370 802 B1**
(45) Veröffentlichungstag und Bekanntmachung des Hinweises auf die Patenterteilung: **06.05.2026**
(21) Anmeldenummer: 23701341.2
(22) Anmeldetag: 18.01.2023
(51) Int. Cl.: F15B 1/24, F15B 21/041, B03C 1/033, G01N 15/06, B03C 1/28, G01N 33/28

(54) **HYDROSPEICHER**
HYDRAULIC ACCUMULATOR
ACCUMULATEUR HYDRAULIQUE

(30) Priorität: 01.02.2022 DE 102022000382
(43) Veröffentlichungstag der Anmeldung: 22.05.2024
(73) Patentinhaber: Hydac Technology GmbH, 66280 Sulzbach/Saar (DE)
(72) Erfinder: WEBER, Norbert, 66125 Saarbrücken (DE)
(74) Vertreter: Bartels und Partner, Patentanwälte
(86) Internationale Anmeldenummer: PCT/EP2023/051122
(87) Internationale Veröffentlichungsnummer: WO 2023/147997

(56) Entgegenhaltungen:
- WO-A1-2023/138874
- CN-A- 111 396 379
- DE-A1- 10 320 799
- DE-U- 1 900 273
- US-A- 3 193 815
- US-A1- 2006 054 402
- US-A1- 2018 045 092

## Beschreibung

Die Erfindung betrifft einen Hydrospeicher, insbesondere in Form eines Kolbenspeichers, mit den Merkmalen im Oberbegriff von Anspruch 1.

Hydrospeicher, wie (hydropneumatische) Kolbenspeicher, kommen in Hydrauliksystemen zum Einsatz, um bestimmte Volumina unter Druck stehender Flüssigkeit, wie Hydrauliköl, aufzunehmen und bei Bedarf an das System zurückzugeben. Bei den üblicherweise eingesetzten hydropneumatischen Kolbenspeichern, bei denen der Kolben den ölseitigen Flüssigkeitsraum von dem ein Arbeitsgas, wie Stickstoffgas, aufnehmenden, im Speichergehäuse eingeschlossenen Speicherraum trennt, verändert sich die Position des Kolbens im Betrieb des Hydrospeichers, sodass der Hydrospeicher beim Anstieg des Drucks Hydrauliköl aufnimmt, wobei dabei gleichzeitig das Arbeitsgas im anderen Fluid- oder Speicherraum komprimiert wird. Bei sinkendem Druck dehnt sich das insoweit verdichtete Gas wieder aus und verdrängt dabei gespeichertes Hydrauliköl zurück in den hydraulischen Arbeitskreislauf. Durch die sich hierdurch im Betrieb ergebenden Veränderungen der Volumina der Arbeitsräume ergibt sich jeweils eine entsprechende Axialbewegung des Kolbens innerhalb des Speichergehäuses.

Insoweit sind dahingehende Hydrospeicher im Rahmen des Betriebs hydraulischer Einrichtungen, wie beispielsweise Arbeitszylinder, an den hydraulischen Arbeitskreislauf angeschlossen, der grundsätzlich zur Abreinigung von partikulärer Verschmutzung aus einem Arbeitsfluid, wie dem Hydrauliköl, über Filtereinrichtungen mit Filterelementen verfügt, die im Bedarfsfall gegen Neuelemente getauscht werden können. Trotz dieser Filtereinrichtungen ist nicht ausgeschlossen, dass Verschmutzungspartikel auf die Reinseite des Fluids gelangen und dann im Hydrospeicher angekommen zu Beschädigungen am dahingehenden Speicher und seinen Komponenten führen. Auch sind Filterelemente vom Durchflussvermögen her limitiert, so dass diese bei sehr hohen Volumenströmen und damit einhergehenden hohen Fluiddrücken nicht immer einsetzbar sind. Insbesondere beim Einsatz von Kolbenspeichern kann die Partikelverschmutzung ungewollt in das Dichtsystem des Trennkolbens gelangen, was zum Versagen des Speichers und damit in Verbindung stehender hydraulischer Einrichtungen führen kann. Da die auftretende Partikelverschmutzung häufig durch Abrieb an den hydraulischen Einrichtungen entsteht, sind diese regelmäßig metallischer Natur und insbesondere im mechanischen Versagensfall können solche auftretenden Partikel auch durchaus eine Größe aufweisen, dass Dichteinrichtungen am Trennkolben mit ihrem Elastomermaterial undicht oder gar zerstört werden.

Durch DE 10 2016 007 798 A1 ist ein hydropneumatischer Kolbenspeicher bekannt, mit einem Speichergehäuse, das ein eine Längsachse definierendes Zylinderrohr aufweist, das an beiden Enden durch jeweils einen Gehäusedeckel hermetisch geschlossen ist und in dem ein Kolben als Trennelement längsverfahrbar geführt ist, der im Gehäuse einen Fluidraum für ein kompressibles Fluid, wie ein Arbeitsgas, von einem weiteren Fluidraum für ein inkompressibles Fluid, wie Hydrauliköl, trennt, und mit einer die Position des Kolbens im Gehäuse berührungslos ermittelnden Wegmesseinrichtung, die ein nichtmagnetisches Messrohr aufweist, das sich durch eine im Kolben gebildete Durchführung entlang der Längsachse von einem Gehäusedeckel zum anderen Gehäusedeckel hin erstreckt und gegen den Innenraum des Gehäuses abgedichtet ist. Im Rohr selbst ist ein Positionsgeber verschiebbar geführt, der durch zwischen ihm und dem Kolben wirkende Magnetkraft im Messrohr den Kolbenbewegungen nachfolgt. Zur Erzeugung der die Folgebewegungen des Positionsgebers im Messrohr erzwingenden Magnetkraft ist ein Permanentmagnet am Kolben vorgesehen. Dieser fortlaufend sich im Betrieb des Speichers zusammen mit dem Trennkolben bewegende Permanentmagnet ist im Fluidraum mit dem kompressiblen Arbeitsgas aufgenommen.

Durch DE 41 16 482 A1 ist ein Verfahren nebst Vorrichtung zum Messen des Druckes eines Arbeitsgases in einem Gasdruckspeicher bekannt, der an einen hydraulischen Arbeitskreislauf anschließbar ist und bei dem das Arbeitsgas über ein Trennelement in Form einer elastomeren Speicherblase von der Betriebs- oder Arbeitsflüssigkeit getrennt ist. Bei einer vorgebbaren Lage der Speicherblase wird der ihr in dieser Lage zuordenbare Gasdruck mittels eines fluidseitig angeordneten Druckwertaufnehmers gemessen, wofür die Position eines Tellerventils des Speichers mittels einer Überwachungseinrichtung überwacht ist. Hierfür weist das Tellerventil in einem Fluidanschluss des Speichers ein Schaltglied auf mit einem Permanentmagneten und der zugehörige Sensor besteht aus einem mittels des Magneten betätigbaren Schalter oder nutzt den sogenannten Hall-Effekt aus, sobald das Schaltglied in Abhängigkeit von der Betätigungsstellung des Tellerventiles an dem dahingehenden Sensor vorbeigeführt ist und diesen auslöst.

Der insoweit sich während des Betriebes des Speichers ständig hin und her bewegende, würfelförmige Magnet ist ebenso wenig wie der Ringmagnet auf der Gasseite des Speichergehäuses geeignet, einer im Speicher auf der Flüssigkeitsseite auftretenden Partikelverschmutzung wirksam entgegentreten zu können.

Die DE 1 900 273 U beschreibt einen Hydrospeicher, insbesondere in Form eines Kolbenspeichers, mit den Merkmalen im Oberbegriff von Anspruch 1 mit einem Trennelement, das in einem Speichergehäuse angeordnet zwei Fluidräume, insbesondere einen abgeschlossenen Speicherraum mit einem Arbeitsgas von einem Flüssigkeitsraum mit einer Betriebsflüssigkeit, wie Hydrauliköl, fluiddicht voneinander trennt, wobei ein Fluidanschluss mit einem der Fluidräume fluidführend verbunden ist, wobei in einem Teil des Speichergehäuses, das den Fluidanschluss aufweist, eine magnetfelderzeugende Einrichtung derart aufgenommen ist, dass magnetisierbare Partikel in abreinigender Weise aus dem zwischen dem Teil des Speichergehäuses und dem Trennelement jeweils befindlichen Fluid in Richtung der magnetfelderzeugenden Einrichtung an dieser abscheidbar sind, wobei das Teil des Speichergehäuses mit der magnetfelderzeugenden Einrichtung ein Gehäusedeckel ist, der ein rohrartiges Wandteil des Speichergehäuses an einem Ende abschließt, das einer Flüssigkeitsseite zugewandt ist, wobei der Fluidanschluss der Flüssigkeitsseite mittig und koaxial zur Längsachse des Speichergehäuses verlaufend den Gehäusedeckel durchgreift, wobei die magnetfelderzeugende Einrichtung demgegenüber außermittig im Gehäusedeckel angeordnet ist, und wobei der Gehäusedeckel in Richtung des Trennelementes eine eben verlaufende Oberseite aufweist.

Weitere Hydrospeicher gehen aus der CN 111 396 379 A und der DE 103 20 799 A1 hervor.

Magnetfelderzeugende Einrichtungen wurden in der US 2006/0054402 A1 und der US 2018/0045092 A1 offenbart.

Eine Detektoreinrichtung wird durch die US 3 193 815 aufgezeigt.

Ausgehend von diesem Stand der Technik liegt daher der Erfindung die Aufgabe zugrunde, die bekannten Hydrospeicherlösungen dahingehend weiter zu verbessern, dass auch im Fall des Auftretens von metallischer Partikelverschmutzung der Versagensfall ausgeschlossen werden kann.

Eine dahingehende Aufgabe löst ein Hydrospeicher mit den Merkmalen des Patentanspruches 1.

Gemäß dem Kennzeichen von Anspruch 1 ist vorgesehen, dass gegenüber der Oberseite vertieft in einer Aufnahme die gesamte magnetfelderzeugende Einrichtung im Gehäusedeckel angeordnet ist.

Durch die vertiefte Anordnung der magnetfelderzeugenden Einrichtung im Gehäusedeckel, als Teil des Speichergehäuses, ist ein Sammelraum gebildet, in den etwaig auftretende Partikel sich abscheiden können, ohne auf der Oberseite des Gehäusedeckels liegen zu bleiben, was ansonsten zu Beschädigungen führen könnte, sofern das kolbenartige Trennelement auf die Oberseite des Gehäusedeckels im Rahmen des üblichen Betriebes schlagartig auftreffen sollte.

Dadurch, dass in einem Teil des Speichergehäuses, das den Fluidanschluss aufweist, eine magnetfelderzeugende Einrichtung derart aufgenommen ist, dass magnetisierbare Partikel in abreinigender Weise aus dem zwischen dem Teil des Speichergehäuses und dem Trennelement jeweils befindlichen Fluid in Richtung der magnetfelderzeugenden Einrichtung an dieser abscheidbar sind, ist eine Möglichkeit geschaffen, ein derart starkes, ortsfest angeordnetes Magnetfeld im Fluidbereich des Speichergehäuses entstehen zu lassen, dass magnetisierbare, insbesondere metallische Partikel, sofern sie auf die Flüssigkeitsseite des Speichers gelangen sollten, insoweit an zentraler Stelle gesammelt und damit aus dem Fluid entfernt zu keinen Schäden führen, insbesondere nicht zu Schäden am Trennelement, wie einem Trennkolben. Insbesondere kann die Partikelverschmutzung nicht auf die Abdichtungsseite des Trennkolbens mit seinen Dichtringen und Führungsbändern eines hydropneumatischen Kolbenspeichers gelangen, was ansonsten neben Abdichtungsproblemen auch dazu führen kann, dass der Trennkolben am Inneren des Speichergehäuses durch Reibung "frisst", mit der Folge, dass der Trennkolben keine Bewegungen mehr ausführen kann, was insgesamt den Hydrospeicher unbrauchbar werden lässt. Insbesondere bei der Beherrschung von Fluidströmen, wo Filtereinrichtungen ihre Grenzen finden, ist dergestalt eine verlässliche Abreinigung von metallischen, magnetisierbaren Partikeln aus dem Fluidstrom mittels der Magnetabscheidung des Hydrospeichers erreicht.

Das Teil des Speichergehäuses mit der magnetfelderzeugenden Einrichtung ist ein Gehäusedeckel, der ein rohrartiges Wandteil des Speichergehäuses an einem Ende abschließt, das einer Flüssigkeitsseite des Hydrospeichers zugewandt ist. Dergestalt lässt sich in kostengünstiger Weise in einem ersten Arbeitsschritt der Gehäusedeckel in das zylindrische Wandteil des Speichergehäuses einsetzen und anschließend wird in einem zweiten Arbeitsschritt die magnetfelderzeugende Einrichtung in den Gehäusedeckel eingebracht.

Der Fluidanschluss der Flüssigkeitsseite durchgreift mittig und koaxial zur Längsachse des Speichergehäuses verlaufend den Gehäusedeckel und die magnetfelderzeugende Einrichtung ist demgegenüber außermittig im Gehäusedeckel angeordnet. Dergestalt lässt sich in platzsparender Weise sowohl der Fluidanschluss als auch die magnetfelderzeugende Einrichtung im Gehäusedeckel unterbringen. Bei einer weiteren bevorzugten Ausführungsform wäre es denkbar, dass mehrere magnetfelderzeugende Einrichtungen in den Gehäusedeckel eingebracht sind. In jedem Fall ist aber die magnetfelderzeugende Einrichtung in der Nähe des Fluidanschlusses und damit im Zuströmbereich des Fluids untergebracht, sodass unmittelbar eine Partikelentnahme aus dem Fluid zeitnah ermöglicht ist.

Bei einer weiteren besonders bevorzugten Ausführungsform des erfindungsgemäßen Hydrospeichers ist vorgesehen, dass die magnetfelderzeugende Einrichtung aus einem Permanentmagneten besteht. Es besteht aber auch die Möglichkeit, die dahingehende Einrichtung aus einem bestrombaren Magneten zu bilden, was dann eine entsprechende Energiezufuhr zu dem Hydrospeicher voraussetzt.

Vorzugsweise ist dabei der Permanentmagnet als Magnetschraube ausgebildet, in einem nicht-magnetischen Einsatz festlegbar, insbesondere einschraubbar, und vorzugsweise ist die Magnetschraube zusammen mit diesem Einsatz in die zugehörige Aufnahme im Gehäusedeckel einsetzbar, insbesondere einschraubbar. Dergestalt lässt sich in bevorzugter Montageausgestaltung der Einsatz mit der Magnetschraube von einer Unterseite des Gehäusedeckels, die dem Trennelement abgewandt ist, in die Aufnahme einsetzen. Dergestalt lässt sich im Rahmen einer Wartung die Magnetschraube austauschen und/oder von magnetisierbaren Partikelverunreinigungen befreien.

Bei einer weiteren bevorzugten Ausführungsform des erfindungsgemäßen Hydrospeichers ist vorgesehen, dass durch eine erste Vertiefung zwischen dem Einsatz und der ebenen Unterseite des Gehäusedeckels und durch eine zweite Vertiefung zwischen der Magnetschraube und der der Magnetschraube gegenüberliegenden freien Stirnseite des Einsatzes, ein gestufter Aufnahmeraum geschaffen ist, dessen freier Durchmesser sich in Richtung der ebenen Unterseite des Gehäusedeckels in Stufen erweitert. Durch die beiden genannten Vertiefungen entsteht so ein vergrößerter Einbringraum, was die Montage von Einsatz nebst Magnetschraube in den unteren Gehäusedeckel erleichtert sowie das spätere Ausbringen der Magnetschraube zu Reinigungszwecken.

Bei einer weiteren besonders bevorzugten Ausführungsform des erfindungsgemäßen Hydrospeichers ist vorgesehen, dass die Magnetschraube eine Detektiereinrichtung aufweist, die bei Überschreiten einer vorgebbaren Menge von magnetisierbarer Partikelverschmutzung anspricht. Hierfür weist vorzugsweise die Detektiereinrichtung eine Spannungsquelle auf, die an ein als Pol ausgebildetes Magnetteil der Magnetschraube angeschlossen ist, das zu einem Leiter als dem weiteren Pol auf Abstand gehalten, an eine Auswerteeinheit ein Signal weiterleitet, sobald mittels der magnetisierbaren Partikelverschmutzung eine elektrische Verbindung zwischen Magnetteil und Leiter der Magnetschraube hergestellt ist. Hierbei kann ein Signal- Schwellenwert bestimmt werden und erst bei dessen Überschreiten wird eine Partikelverschmutzung als derart störend signalisiert, dass eine Abreinigung der Magnetschraube im Rahmen von Wartungsarbeiten als notwendig durchgeführt wird.

In den üblicherweise geschlossen ausgeführten Hydraulikkreisläufen können metallische Partikel zur Beschädigung von empfindlichen Bauteilen wie Ventilen, Kolbenspeicherdichtungen etc. führen. Gerade in der Hydraulik von Baumaschinen kann durch Ausfall oder Verschleiß von aktiven Elementen, wie Ventilen, Zylindern, Aktuatoren etc. ein metallischer Abrieb erzeugt werden, der dann durch den Kreislauf bis zu den Hydrospeichern "gespült" wird. Dahingehende Hydrospeicher, insbesondere Kolbenspeicher, werden oft in Bremssystemen eingesetzt, sodass durch Zerstörung der Kolbendichtung aufgrund der Partikelverschmutzung die Funktionsweise der Bremse eingeschränkt wird oder sogar vollständig in Wegfall gerät. Durch den Einsatz von magnetischen Elementen, wie handelsübliche Magnetschrauben, können solche Partikel aus dem Hydraulikfluid herausgezogen und dadurch die Lebensdauer der Bauteile des hydraulischen Kreislaufs signifikant verlängert werden. Der Einsatz von Magnetschrauben hat jedenfalls den Vorteil, dass die jeweilige Schraube zum Entsorgen der Partikel im Rahmen von Wartungsarbeiten von Hand herausgedreht und ersetzt oder abgereinigt werden kann. Mit Hilfe des zuletzt angesprochenen Verschmutzungsschalters als Detektiereinrichtung kann die Verschmutzung angezeigt werden, indem die magnetisierbaren, metallischen Partikel den zugehörigen Strom- oder Schaltkreis unter Abgabe eines Signals schließen.

Im Folgenden wird der erfindungsgemäße Hydrospeicher anhand eines Ausführungsbeispiels nach der Zeichnung näher erläutert. Dabei zeigen in prinzipieller und nicht maßstäblicher Darstellung die
- Fig. 1: einen Längsschnitt durch den Hydrospeicher;
- Fig. 2: eine stirnseitige Unteransicht auf den Hydrospeicher nach der Fig. 1; und
- Fig. 3: die Verwendung eines Verschmutzungsschalters zum Indizieren von Verschmutzungen bei einer Magnetschraube, wie sie bei einer Hydrospeicherlösung nach den Fig. 1 und 2 Anwendung findet.

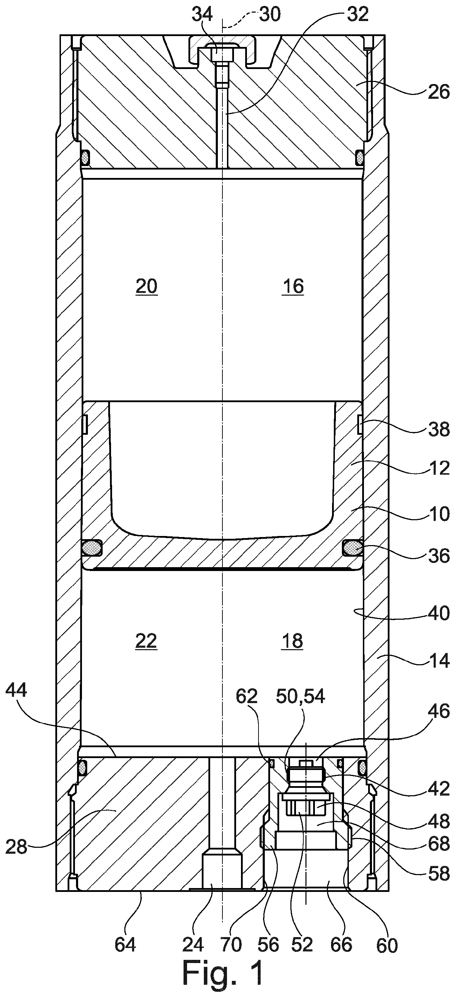

Der in der Fig. 1 dargestellte erfindungsgemäße Hydrospeicher, in Form eines sogenannten Kolbenspeichers, weist als Trennelement 10 einen Trennkolben 12 auf, der in einem Speichergehäuse 14 angeordnet zwei Fluidräume 16, 18 voneinander trennt. Der in Blickrichtung auf die Fig. 1 gesehen obere Fluidraum 16 bildet einen abgeschlossenen Speicherraum 20 für die Aufnahme eines Arbeitsgases aus, wie beispielsweise Stickstoffgas. Der untere Fluidraum 18 bildet einen Flüssigkeitsraum 22 aus zwecks Aufnahme einer Betriebsflüssigkeit, wie beispielsweise Hydrauliköl. Der dahingehende Fluidraum 18 respektive der Flüssigkeitsraum 22 ist mit einem Fluidanschluss 24 versehen, über den der Speicher, was nicht näher dargestellt ist, an einen hydraulischen Arbeitskreislauf üblicher Art angeschlossen werden kann.

Das Speichergehäuse 14 ist in der Art eines runden Hohlzylinders oder Zylinderrohres ausgebildet, das an seinen beiden freien Enden durch jeweils einen eingeschraubten Gehäusedeckel 26, 28 dicht verschlossen ist, zwischen denen der Trennkolben 12 koaxial entlang der Gehäuselängsachse 30 frei verfahrbar geführt ist.

Der obere Gehäusedeckel 26 weist einen durchgehenden Fluidkanal 32 auf, der gemäß der Darstellung nach der Fig. 1 von einer Verschlussschraube 34 verschlossen ist. Über die dahingehende Anordnung 32, 34 lässt sich im Bedarfsfall der Speicher von Arbeitsgas im Raum 16, beispielsweise zu Wartungszwecken entleeren, aber auch bei fehlendem Arbeitsgas den Raum 16 befüllen. Der Trennkolben 12 selbst ist als sogenannter Hohlkolben ausgeführt zwecks Vergrößern des wirksamen Gasvolumens auf Seiten des Fluidraumes 16. Außenumfangsseitig ist der Trennkolben 12 über mindestens einen Dichtring 36 sowie mindestens ein Führungsband 38 entlang der Innenseite 40 des zylindrischen Speichergehäuses 14 geführt. Im Rahmen der Realisierung praktischer Ausführungsformen können mehrere solche Dichtringe und Führungsbänder auch in Kombination miteinander am Außenumfang des Trennkolbens 12 angebracht sein.

Der bisherige Aufbau eines solchen Hydrospeichers, hier in Form eines Kolbenspeichers, ist üblich und wird daher nicht mehr näher detailliert beschrieben und nur noch insoweit als es zum Verständnis der Erfindung notwendig ist. So ist in dem unteren Gehäusedeckel 28 als Teil des Speichergehäuses 14, das den Fluid- oder Flüssigkeitsanschluss 24 aufweist, derart eine magnetfelderzeugende Einrichtung 42 aufgenommen, dass magnetisierbare, insbesondere metallische, Partikel in abreinigender Weise aus dem zwischen dem unteren Gehäusedeckel 28 als Teil des Speichergehäuses 14 und dem Trennelement 10 in Form des Trennkolbens 12 jeweils befindlichen Fluid in Richtung der magnetfelderzeugenden Einrichtung 42, an dieser abscheidbar sind. Das im Fluidraum 18 bzw. im Flüssigkeitsraum 22 befindliche Fluid nimmt je nach Betriebszustand des Kolbenspeichers unterschiedliche Volumina ein, wobei insbesondere in Blickrichtung auf die Fig. 1 gesehen, bei einer Abwärtsbewegung des Trennelementes 10 respektive des Trennkolbens 12 in Richtung des unteren Gehäusedeckels 28, das Fluid aus dem Fluidanschluss 24 zurück in den hydraulischen Kreislauf gelangt und etwaig im Raum 18, 22 befindliche magnetisierbare Partikel werden in Richtung der magnetfelderzeugenden Einrichtung 42 im Boden des Gehäusedeckels 28 gelenkt und dort gesammelt. Aber auch bereits bei Eintritt des Fluids über den Fluidanschluss 24 in den Raum 18, 22 ist die Magnetkraft der magnetfelderzeugenden Einrichtung 42 derart stark, dass etwaige magnetisierbare Partikel wirksam angezogen und an der Einrichtung 42 gesammelt werden können.

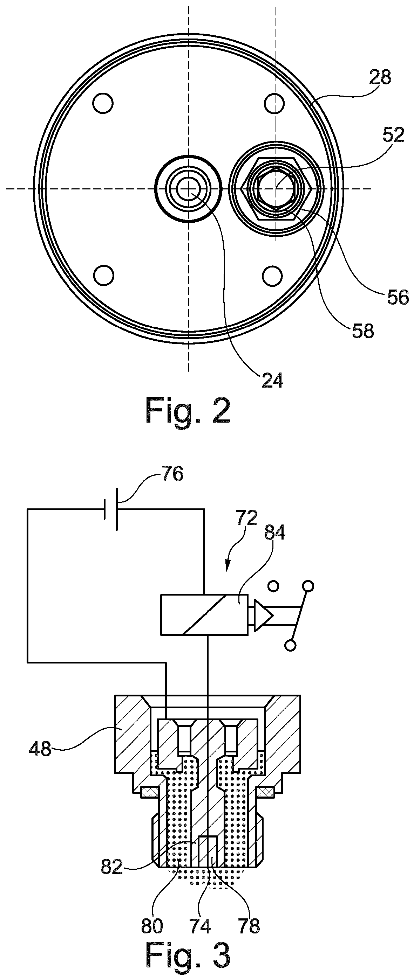

Wie sich insbesondere aus den Fig. 1 und 2 ergibt, ist der Fluidanschluss 24, der einer Flüssigkeitsseite des Hydrospeichers mit zugeordneter Verrohrung eines Hydraulikkreislaufes (nicht dargestellt) zugehörig ist, mittig und koaxial zur Längsachse 30 des Speichergehäuses 14 verlaufend im unteren Gehäusedeckel 28 und diesen vollständig durchgreifend integriert. Demgegenüber ist die magnetfelderzeugende Einrichtung 42 außer mittig im Gehäusedeckel 28 angeordnet. Dergestalt kann ungehindert das Fluid über den Fluidanschluss 24 in den Raum 18, 22 ein- und aus diesem wieder heraus in Richtung des Hydraulikkreislaufes ausströmen, ohne dass dieser freie Strömungsfluss durch die ein Magnetfeld erzeugende, außermittig liegende Einrichtung 42 behindert wäre, die insoweit nur dem Abscheiden von Magnetpartikeln aus dem zugehörigen Fluidstrom dient. Im Bedarfsfall können mehrere Einrichtungen 42 der genannten Art um die Gehäuselängsachse 30 herumgruppiert im Gehäusedeckel 28 aufgenommen sein.

Wie sich des Weiteren aus der Fig. 1 ergibt, weist der Gehäusedeckel 28 in Richtung des Trennelementes 10 eine eben verlaufende Oberseite 44 auf, gegenüber der vertieft in einer zylindrischen Aufnahme 46 die magnetfelderzeugende Einrichtung 42 im Gehäusedeckel 28 angeordnet ist. Die magnetfelderzeugende Einrichtung 42 besteht bevorzugt aus einem Permanentmagneten; es besteht aber auch die Möglichkeit, einen bestrombaren Elektromagneten an dieser Stelle im Gehäusedeckel 28 auf der Flüssigkeitsseite zu verwenden. Im vorliegenden Fall ist der Permanentmagnet jedoch als sogenannte Magnetschraube 48 ausgebildet, sprich also aus einer üblichen Schraube mit Außengewinde 50 und Schraubenkopf 52, die vollständig aus magnetischem Material besteht oder ein solches Material als Werkstoffbestandteil aufweist. Mit ihrem Außengewinde 50 ist die Magnetschraube 48 in einem zugehörigen Innengewinde 54 eines Einsatzes 56 festgelegt, der mit seinem kopfseitig in einer Verbreiterung angebrachten Außengewinde 58 in ein zugehöriges Innengewinde 60 im unteren Gehäusedeckel 28 festlegbar ist. Wie ferner die Fig. 1 zeigt, verfügt der Einsatz 56 an seinem freien, stirnseitigen Endbereich in Richtung des Fluidraumes 18 über eine Abdichtung in Form eines Dichtringes 62. Der Einsatz 56 mit der Magnetschraube 48 ist von Seiten der eben verlaufenden Unterseite 64 des unteren Gehäusedeckels 28, die dem Trennelement 10 abgewandt ist, in die Aufnahme 46 eingesetzt. Dabei ist eine erste Vertiefung 66 zwischen dem Einsatz 56 und der ebenen Unterseite 64 des Gehäusedeckels 28 gebildet und eine weitere zweite Vertiefung 68 ist zwischen der Magnetschraube 48 und der der Magnetschraube 48 gegenüberliegenden freien Stirnseite des Einsatzes 56 vorhanden. Derart ist ein gestufter Aufnahmeraum 70 geschaffen, dessen freier Durchmesser sich in Richtung der ebenen Unterseite 64 des Gehäusedeckels 28 in Stufen erweitert und dergestalt ist in besonders montagefreundlicher Weise das Ein- und Ausschrauben der Magnetschraube 48 in bzw. aus dem Einsatz 56 heraus ermöglicht. Dergestalt kann bei flüssigkeitsentleertem Hydrospeicher die Magnetschraube 48 zusammen mit den aufgefangenen magnetischen Partikeln aus dem Gehäusedeckel 28 entfernt und nach Abreinigen von den Partikeln wieder in ihre die Partikel aus der Flüssigkeit anziehende und fangende Stellung gebracht werden.

Wie insbesondere die Fig. 3 zeigt, kann bei einer geänderten Ausführungsform der Magnetschraube 48 diese mit einer als Ganzes mit 72 bezeichneten Detektiereinrichtung ausgestattet sein, die bei Überschreiten einer vorgebbaren Menge 74 von magnetisierbarer Partikelverschmutzung anspricht.

Dahingehend weist die Detektiereinrichtung 72 eine übliche Spannungsquelle 76 auf, die an ein als Pol ausgebildetes Magnetteil 78 der Magnetschraube 48 stromführend angeschlossen ist. Das Magnetteil 78 ist aus einem zylindrischen Permanentmagneten gebildet, der stirnseitig in die Magnetschraube 48 in eine Ausnehmung derselben eingelassen ist. In konzentrischer Anordnung umfasst ein hohlzylindrischer, elektrischer Leiter 80 das innere Magnetteil 78, wobei zur Vermeidung eines Kurzschlusses zwischen die beiden Komponenten 78, 80 ein nicht leitfähiges Hülsenstück als Trennhülse 82 eingebracht ist und dergestalt die beiden Komponenten 78, 80 elektrisch voneinander entkoppelt. Ansonsten ist die Magnetschraube 48 als Ganzes wiederum über ihr Außengewinde 50 in das zugehörige Innengewinde 54 des Einsatzes 56 im Deckelteil 28 einschraubbar.

In den Strompfad zwischen der Spannungsquelle 76 und dem inneren Magnetteil 78 ist ein Relais 84 geschaltet, das als Anzeige dient. Sobald sich am Magnetteil 78 der Magnetschraube 48 eine solche Menge 74 magnetisierbarer Partikel angesammelt hat, dass die Trennhülse 82 in Richtung des elektrischen Leiters 80 mit der Partikelverschmutzung überbrückt ist, kommt es zu einer elektrischen Verbindung zwischen Magnetteil 78 und Leiter 80 und in der Folge spricht das Relais 84 an, das geschaltet eine Anzeige für den dahingehend erreichten Grad an Partikelverschmutzung an der Magnetschraube 48 an eine Bedienperson weitergibt. Die Bedienperson würde dann im Rahmen der Wartung und zum Abreinigen der Magnetschraube 48 an ihrer freien Stirnseite diese aus dem Einsatz 56 im Gehäusedeckel 28 entfernen und nach Abreinigen wieder einsetzen. Dergestalt ist über die Einrichtung nach der Fig. 3 eine Art Verschmutzungsschalter zum Indizieren von Verschmutzung an der Magnetschraube 48 realisiert. Bis auf den Permanentmagneten 78 und den elektrischen Leiter 80 sind die sonstigen Teile der Magnetschraube 48 im vorliegenden Fall, vorzugsweise aus nichtmetallischem Werkstoff gebildet, beispielsweise aus einem geeigneten Kunststoffmaterial.

Gerade bei Baumaschinen kann durch Ausfall oder Verschleiß von aktiven Elementen, wie Ventilen, Arbeitszylindern respektive Aktuatoren metallischer Abrieb erzeugt werden, der dann durch den Kreislauf bis zu den Hydrospeichern "gespült" wird. Dahingehende Hydrospeicher werden regelmäßig auch in Bremssystemen von dahingehenden Arbeitsmaschinen, wie Land- oder Baumaschinen, eingesetzt, wodurch Zerstörung der Dichtung des Trennkolbens 12 durch metallische Partikel die Funktionsweise eingeschränkt oder sogar komplett verhindert wird.

Durch den Einsatz von magnetischen Elementen, wie hier in Form einer Magnetschraube 48, die gemäß der Ausführungsform nach der Fig. 3 auch nur in ihrem vordersten freien Stirnbereich einen Permanentmagneten 78 aufweisen kann, können solche magnetisierbaren metallischen Partikel aus dem Hydraulikfluid herausgezogen und dadurch die Lebensdauer von Bauteilen verlängert werden. Durch das "Herausfischen" solcher Partikel mittels magnetfelderzeugender Einrichtung 42 im sich ändernden Kammervolumen zwischen Trennelement 10 und unterem Gehäusedeckel 28, wird in geschlossenen Hydraulikkreisläufen verhindert, dass die dahingehenden metallischen Partikel den Hydrospeicher verlassend zum Beschädigen von empfindlichen Bauteilen, wie Ventilen, Kolbenspeicher, Dichtungen, etc. führen können, so dass insgesamt alle Komponenten eines Hydraulikkreislaufes vor solchen Beeinträchtigungen geschützt sind. Dies hat so keine Entsprechung im Stand der Technik.

Die erfindungsgemäße Abscheideeinrichtung von magnetisierbaren Partikeln braucht auf Kolbenspeicherlösungen, wie vorstehend aufgezeigt, nicht eingeschränkt zu sein, sondern kann vielmehr auch bei anderen Speicherlösungen mit einem Trennelement eingesetzt werden, wie Blasenspeichern, Membranspeichern und Balgspeichern. Ferner kann im Bedarfsfall in den Fluidanschluss 24 auch ein Ventil eingesetzt sein, wie beispielsweise ein für Blasenspeicher üblicher Bauart eingesetztes Tellerventil, ohne die Wirkung der magnetfelderzeugenden Einrichtung 42 integriert im Gehäusedeckel 28 zu beeinträchtigen.

## Patentansprüche

1. Hydrospeicher, insbesondere in Form eines Kolbenspeichers, mit einem Trennelement (10), das in einem Speichergehäuse (14) angeordnet zwei Fluidräume (16, 18), einen abgeschlossenen Speicherraum (20) mit einem Arbeitsgas von einem Flüssigkeitsraum (22) mit einer Betriebsflüssigkeit, wie Hydrauliköl, fluiddicht voneinander trennt, wobei ein Fluidanschluss (24) mit dem Flüssigkeitsraum (22) fluidführend verbunden ist, wobei in einem Teil des Speichergehäuses (14), das den Fluidanschluss (24) aufweist, eine magnetfelderzeugende Einrichtung (42) derart aufgenommen ist, dass magnetisierbare Partikel in abreinigender Weise aus der zwischen dem Teil des Speichergehäuses (14) und dem Trennelement (10) befindlichen Flüssigkeit in Richtung der magnetfelderzeugenden Einrichtung (42) an dieser abscheidbar sind, wobei das Teil des Speichergehäuses (14) mit der magnetfelderzeugenden Einrichtung (42) ein Gehäusedeckel (28) ist, der ein rohrartiges Wandteil des Speichergehäuses (14) an einem Ende abschließt, das der Flüssigkeitsseite zugewandt ist, wobei der Fluidanschluss (24) der Flüssigkeitsseite mittig und koaxial zur Längsachse (30) des Speichergehäuses (14) verlaufend den Gehäusedeckel (28) durchgreift, wobei die magnetfelderzeugende Einrichtung (42) demgegenüber außermittig im Gehäusedeckel (28) angeordnet ist, und wobei der Gehäusedeckel (28) in Richtung des Trennelementes (10) eine eben verlaufende Oberseite (44) aufweist,
**dadurch gekennzeichnet,**
**dass** gegenüber der Oberseite (44) vertieft in einer Aufnahme (46) die gesamte magnetfelderzeugende Einrichtung (42) im Gehäusedeckel (28) angeordnet ist.

2. Hydrospeicher nach Anspruch 1, **dadurch gekennzeichnet, dass** die magnetfelderzeugende Einrichtung (42) aus einem Permanentmagneten besteht.

3. Hydrospeicher nach Anspruch 2, **dadurch gekennzeichnet, dass** der Permanentmagnet als Magnetschraube (48) ausgebildet, in einem nicht-magnetischen Einsatz (56) festlegbar, insbesondere einschraubbar, ist und vorzugsweise zusammen mit dem Einsatz (56) in die zugehörige Aufnahme (46) im Gehäusedeckel (28) einsetzbar, insbesondere einschraubbar, ist.

4. Hydrospeicher nach Anspruch 3, **dadurch gekennzeichnet, dass** der Einsatz (56) mit der Magnetschraube (48) von der Unterseite (64) des Gehäusedeckels (28), die dem Trennelement (10) abgewandt ist, in die Aufnahme (46) eingesetzt ist.

5. Hydrospeicher nach Anspruch 3 oder 4, **dadurch gekennzeichnet, dass** durch eine erste Vertiefung (66) zwischen dem Einsatz (56) und der ebenen Unterseite (64) des Gehäusedeckels (28) und durch eine zweite Vertiefung (68) zwischen der Magnetschraube (48) und der der Magnetschraube (48) gegenüberliegenden freien Stirnseite des Einsatzes (56), ein gestufter Aufnahmeraum (70) geschaffen ist, dessen freier Durchmesser sich in Richtung der ebenen Unterseite (64) des Gehäusedeckels (28) in Stufen erweitert.

6. Hydrospeicher nach einem der vorstehenden Ansprüche, **dadurch gekennzeichnet, dass** die Magnetschraube (48) eine Detektiereinrichtung (72) aufweist, die bei Überschreiten einer vorgebbaren Menge (74) von magnetisierbarer Partikelverschmutzung anspricht.

7. Hydrospeicher nach Anspruch 6, **dadurch gekennzeichnet, dass** die Detektiereinrichtung (72) eine Spannungsquelle (76) aufweist, die an ein als Pol ausgebildetes Magnetteil (78) der Magnetschraube (48) angeschlossen ist, das zu einem Leiter (80) als weiterem Pol auf Abstand gehalten, an eine Auswerteeinheit (84) ein Signal weiterleitet, sobald mittels der magnetisierbaren Partikelverschmutzung eine elektrische Verbindung zwischen den beiden Polen (78, 80) der Magnetschraube (48) hergestellt ist.

## Claims

1. Hydraulic accumulator, in particular in the form of a piston accumulator, comprising a separating element (10), which is arranged in an accumulator housing (14) and separates two fluid chambers (16, 18) in a fluid-tight manner from one another, a closed accumulator chamber (20) containing a working gas from a liquid chamber (22) containing an operating liquid, such as hydraulic oil, a fluid port (24) being connected in a fluid-conducting manner to the liquid chamber (22), a magnetic-field-generating device (42) being received in a part of the accumulator housing (14) that comprises the fluid port (24) in such a way that magnetisable particles can be separated, in a cleaning manner, from the liquid located between the part of the accumulator housing (14) and the separating element (10) in the direction of the magnetic-field-generating device (42), and deposited on said device,
wherein the part of the accumulator housing (14) containing the magnetic-field-generating device (42) is a housing lid (28) that closes a tubular wall part of the accumulator housing (14) at one end, which faces the liquid side,
wherein the fluid port (24) of the liquid side engages through the housing lid (28), running in the centre and coaxially with respect to the longitudinal axis (30) of the accumulator housing (14),
wherein the magnetic-field-generating device (42) is arranged opposite thereto, off-centred in the housing lid (28), and
wherein the housing lid (28) comprises a flat upper side (44) in the direction of the separating element (10),
**characterised in that**
the entire magnetic-field-generating device (42) is arranged in the housing lid (28), recessed in a receptacle (46) in relation to the upper side (44).

2. Hydraulic accumulator according to claim 1, **characterised in that** the magnetic-field-generating device (42) consists of a permanent magnet.

3. Hydraulic accumulator according to claim 2, **characterised in that** the permanent magnet is configured as a magnetic plug (48), which can be fixed, in particular screwed, in a non-magnetic insert (56), and preferably inserted, or in particular screwed, together with the insert (56), into the corresponding receptacle (46) in the housing lid (28).

4. Hydraulic accumulator according to claim 3, **characterised in that** the insert (56) with the magnetic plug (48) is inserted into the receptacle (46) from the underside (64) of the housing lid (28), which faces away from the separating element (10).

5. Hydraulic accumulator according to either claim 3 or claim 4, **characterised in that**, by means of a first recess (66) between the insert (56) and the flat underside (64) of the housing lid (28) and by means of a second recess (68) between the magnetic plug (48) and the free end face of the insert (56) opposite the magnetic plug (48), a stepped receiving space (70) is created, the free diameter of which widens in stages in the direction of the flat underside (64) of the housing lid (28).

6. Hydraulic accumulator according to any of the preceding claims, **characterised in that** the magnetic plug (48) comprises a detection device (72), which responds if a predefinable quantity (74) of magnetisable particulate contamination is exceeded.

7. Hydraulic accumulator according to claim 6, **characterised in that** the detection device (72) comprises a voltage source (76), which is connected to a magnetic part (78) of the magnetic plug (48) configured as a pole, said magnetic part being spaced apart from a conductor (80) as a further pole and transmitting a signal to an evaluation unit (84) as soon as an electrical connection is established between the two poles (78, 80) of the magnetic plug (48) by means of the magnetisable particulate contamination.

## Revendications

1. Accumulateur hydraulique, en particulier sous la forme d'un accumulateur à piston, comprenant un élément (10) de séparation, qui, disposé dans un carter (14) de l'accumulateur, sépare l'un de l'autre, d'une manière étanche au fluide, deux espaces (16, 18) pour du fluide, un espace (20) d'accumulateur fermé ayant un gaz de travail d'un espace (22) pour du liquide, ayant un liquide de fonctionnement, comme de l'huile hydraulique, dans lequel un raccord (24) pour du fluide communique fluidiquement avec l'espace (22) pour du liquide, dans lequel dans une partie du carter (14) de l'accumulateur, qui a le raccord (24) pour du fluide, est logé un dispositif (42) produisant un champ magnétique, de manière à pouvoir déposer, en direction du dispositif (42) de production d'un champ magnétique sur celui-ci, des particules magnétiques, en mode de nettoyage, du liquide se trouvant entre la partie du carter (14) de l'accumulateur et l'élément (10) de séparation,
dans lequel
la partie du carter (14) de l'accumulateur, ayant le dispositif (42) produisant un champ magnétique, a un couvercle (28) de carter, qui ferme, à une extrémité tournée vers le côté du liquide, une partie de paroi de type tubulaire du carter (14) de l'accumulateur,
dans lequel
le raccord (24) pour du fluide du côté du liquide traverse le couvercle (28) du carter en s'étendant au milieu et coaxialement à l'axe (30) longitudinal du carter (14) de l'accumulateur, dans lequel le dispositif (42) produisant un champ magnétique est disposé en revanche en dehors du milieu dans le couvercle (28) du carter, et
dans lequel
le couvercle (28) du carter a, dans la direction de l'élément (10) de séparation, une face (44) supérieure s'étendant de manière plane,
**caractérisé**
**en ce que**,
enfoncé par rapport à la face (44) supérieure dans un logement (46), l'ensemble du dispositif (42) produisant un champ magnétique est disposé dans le couvercle (28) du carter.

2. Accumulateur hydraulique suivant la revendication 1, **caractérisé en ce que** le dispositif (42) produisant un champ magnétique est constitué d'un aimant permanent.

3. Accumulateur hydraulique suivant la revendication 2, **caractérisé en ce que** l'aimant permanent est constitué sous la forme d'une vis (48) magnétique, peut être fixé dans un insert (56) amagnétique en y étant, en particulier, vissé et peut, de préférence ensemble avec l'insert (56), être inséré en y étant, en particulier, vissé dans le logement (46) lui appartenant dans le couvercle (28) du carter.

4. Accumulateur hydraulique suivant la revendication 3, **caractérisé en ce que** l'insert (56) est, avec la vis (48) magnétique, inséré dans le logement (46) par la face (64) inférieure du couvercle (28) du carter, qui n'est pas tournée vers l'élément (10) de séparation.

5. Accumulateur hydraulique suivant la revendication 3 ou 4, **caractérisé en ce que**, par une première cavité (66) entre l'insert (56) et la face (64) inférieure plane du couvercle (28) du carter et par une deuxième cavité (68) entre la vis (48) magnétique et le côté frontal libre, opposé à la vis (48) magnétique, de l'insert (56), est ménagé un espace (70) étagé de réception, dont le diamètre libre s'agrandit par palier en direction de la face (64) inférieure plane du couvercle (28) du carter.

6. Accumulateur hydraulique suivant l'une des revendications précédentes, **caractérisé en ce que** la vis (48) magnétique a un dispositif (72) de détection, qui réagit, si une quantité (74) pouvant être donnée à l'avance de pollution par des particules magnétisables, est dépassée.

7. Accumulateur hydraulique suivant la revendication 6, **caractérisé en ce que** le dispositif (72) de détection a une source (76) de tension, qui est raccordée à une partie (78) magnétique constituée en pôle de la vis (48) magnétique, qui est maintenue à distance d'un conducteur (80) comme autre pôle, qui achemine un signal à une unité (84) d'évaluation, dès que, au moyen de la pollution de particules magnétisables, une connexion électrique est produite entre les deux pôles (78, 80) de la vis (48) magnétique.
